(19) Europäisches Patentamt
European Patent Office
Office européen des brevets

(11) **EP 3 778 852 B1**

(12) **EUROPEAN PATENT SPECIFICATION**

(45) Date of publication and mention
of the grant of the patent:
**29.07.2026 Bulletin 2026/31**

(21) Application number: **19777400.3**

(22) Date of filing: **20.03.2019**

(51) International Patent Classification (IPC):
***C12R 1/145*** (2006.01)    ***C12M 1/00*** (2006.01)
***C12P 7/06*** (2006.01)

(52) Cooperative Patent Classification (CPC):
**C12M 47/12; C12M 21/12; C12M 43/02; C12P 7/06;**
C12R 2001/145; Y02E 50/10

(86) International application number:
**PCT/JP2019/011905**

(87) International publication number:
**WO 2019/188726 (03.10.2019 Gazette 2019/40)**

(54) **DEVICE FOR MANUFACTURING ORGANIC SUBSTANCE, AND GAS PROCESSING SYSTEM**

VORRICHTUNG ZUR HERSTELLUNG EINER ORGANISCHEN SUBSTANZ UND GASVERARBEITUNGSSYSTEM

DISPOSITIF DE FABRICATION D'UNE SUBSTANCE ORGANIQUE, ET SYSTÈME DE TRAITEMENT DE GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2018 JP 2018060754**

(43) Date of publication of application:
**17.02.2021 Bulletin 2021/07**

(73) Proprietor: **SEKISUI CHEMICAL CO., LTD.**
**Osaka-shi**
**Osaka**
**530-8565 (JP)**

(72) Inventor: **ISHII Tetsuya**
**Tsukuba-shi, Ibaraki 300-4292 (JP)**

(74) Representative: **Ter Meer Steinmeister & Partner**
**Patentanwälte mbB**
**Nymphenburger Straße 4**
**80335 München (DE)**

(56) References cited:
| | |
|---|---|
| EP-A1- 3 199 619 | EP-A2- 2 698 423 |
| WO-A1-2016/043163 | WO-A2-2011/003962 |
| CN-A- 104 893 956 | JP-A- 2016 010 777 |
| JP-A- 2016 010 777 | JP-A- 2016 029 921 |
| JP-A- 2016 029 921 | JP-A- 2017 123 791 |
| JP-A- 2017 123 791 | US-A1- 2009 215 139 |
| US-A1- 2009 239 288 | US-A1- 2014 272 926 |

**Description**

Technical Field

The present invention relates to a device for producing an organic substance and a gas treatment system.

Background Art

These days, gas fermentation by microorganisms (bacteria) is investigated to produce an organic substance (e.g., refer to PTL 1). In the method described in PTL 1, a culture broth after fermentation by a microorganism discharged from a fermentation vat is directly supplied to a purification device to purify the organic substance.
In such a method, however, use of a distillation column as the purification device causes foaming in the distillation column depending on the type of microorganism, which causes difficulty in continuous distillation.

Citation List

Patent Literature

PTL 1: JP 2015-077120 A
JP2016 029921 is directed to a device and a method for producing an organic substance, such as ethanol by microbial fermentation. The apparatus comprises an organic substance synthesis unit, a heating unit, a separation unit and an extraction unit. The heating unit heats the liquid from the organic substance synthesis unit. The separation unit separates solid components from the heated liquid. The extraction unit extracts the organic substance from the liquid from which the solid has been separated. In the apparatus the organic substance is generated by fermenting a gas, e.g. synthesis gas, like $CO_2$, by microorganisms, such as a bacterium, e.g. Clostridium. The extraction part may be obtained the organic substance by distilling the liquid from which the solid component was separated before. The separation device uses filters to separate the solid content from the liquid.
US 2009/215139 is directed to a device for generating ethanol from natural gas solid waste or land fill gas, wherein pressure vaporizes the liquid prior to entering a column. The liquid steam passes through an expansion valve vaporizing the liquid and entering the column. The column separates the ethanol stream into an ethanol depleted bottoms stream and an overhead vapor stream. At least a portion of the first vapor permeate stream returns to the column.

Summary of Invention

Technical Problem

It is an object of the present invention to provide a device for producing an organic substance capable of preventing foaming in a purification section to purify an organic substance and obtaining the organic substance with high yield and a gas treatment system having such a device for producing an organic substance.

Solution to Problem

Such an object is achieved by the technical features of independent claims 1 and 9 with respect to a device for producing an organic substance and a gas treatment system comprising said device.

Advantageous Effects of Invention

According to the present invention, it is possible to prevent foaming in the purification section and thus to increase the yield of the organic substance.

Brief Description of the Drawings

Fig. 1 is a block diagram illustrating an embodiment of a gas treatment system of the present invention.
Fig. 2 is a diagram illustrating a gas treatment system simulated in Example 1.
Fig. 3 is a diagram illustrating a gas treatment system simulated in Example 2.

Description of Embodiments

A device for producing an organic substance and a gas treatment system of the present invention are described below in detail based on preferred embodiments illustrated in the attached drawings.

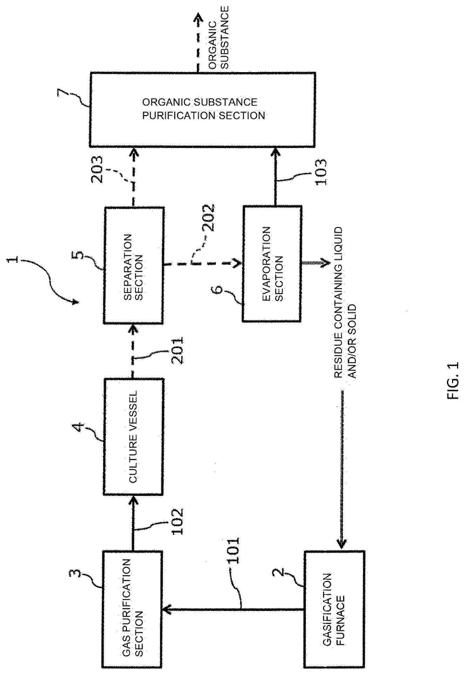

Fig. 1 is a block diagram illustrating an embodiment of a gas treatment system of the present invention.

A gas treatment system 1 illustrated in Fig. 1 includes a gas generation device and a device for producing an organic substance (the device for producing an organic substance of the present invention) connected to the gas generation device. In the present embodiment, the gas generation device is provided with a gasification furnace 2. The gas treatment apparatus includes a gas purification section 3, a fermenter 4, a separation section 5, an evaporation section 6, and an organic substance purification section 7.

The gasification furnace (gas generation section) 2 is a furnace to combust a gas material (carbon source). In the gasification furnace 2, heat treatment of combustion (incomplete combustion) of the gas material generates gas (synthesis gas) containing carbon monoxide as a main component. That is, partial oxidation of the gas material generates gas containing carbon monoxide as a main component. In the gasification furnace 2, gas may be generated by heating the gas material.

In addition to carbon monoxide, the gas thus generated may contain other gas components, such as hydrogen, carbon dioxide, nitrogen, and oxygen, for example, and in particular preferably contains carbon monoxide and hydrogen. Use of such gas allows the microorganism to efficiently generate (produce) an intended organic substance.

In this context, the gas material is not particularly limited as long as the material contains carbon, and examples of the material include coal, biomass materials (wood chips, etc.), petroleum, natural gas, shale gas, various wastes, and the like. Among all, from the perspective of environmental issues, wastes (garbage) collected by local municipalities and various industrial waste agencies is preferably used as the gas material.

Examples of the wastes include: plastic wastes; kitchen garbage; municipal wastes (MSW); waste tires; biomass wastes; household garbage, such as bedding and paper; construction members; and the like, and it is possible to use one type of them singly or two or more types in combination. Use of such a waste allows contribution to a recycling society.

Combustion of the gas material in the gasification furnace 2 may be carried out while air is supplied while it is preferred to be carried out while gas with a higher oxygen concentration than air (high oxygen concentration gas) is supplied.

Use of the high oxygen concentration gas allows elimination of the necessity of heating extra nitrogen contained in a large amount in the air, resulting in higher efficiency of the combustion of the gas material.

The gasification furnace 2 has a gas outlet connected, via a gas line 101, to a gas inlet of the gas purification section 3. The gas generated in the gasification furnace 2 is supplied to the gas purification section 3 by a pump, not shown.

The gas purification section 3 may be configured using one or more types of, for example: a scrubber (water soluble impurity separator) using water, an acidic solution, or an alkaline solution; a gas chiller (moisture separator); a microparticle (soot) separator, such as a cyclone and a bag filter; a desulfurizer (sulfide separator); a low-temperature (cryogenic) separator; a pressure swing adsorption (PSA) separator; a membrane separator; a temperature swing adsorption (TSA) separator; a separator using active carbon; a separator using a copper catalyst or a palladium catalyst; and the like.

The gas purification section 3 has a gas outlet connected, via a gas line 102, to a gas inlet of the fermenter 4. The gas discharged from the gas purification section 3 is supplied to the fermenter 4 by a pump, not shown.

The fermenter 4 is a device to generate an organic substance (valuable) from gas by fermenting action of a microorganism. Specifically, a culture broth from a culture broth tank (not shown) and a microorganism from a reserve culture vessel (not shown) are firstly supplied to the culture vessel 4 to be stored (retained). In this state, gas is supplied to the culture vessel 4 while the culture broth is stirred. The microorganism is thus cultured in the culture broth to generate an organic substance from gas by the fermenting action.

The culture broth is a liquid containing water as a main component and nutrients (e.g., vitamins, phosphoric acid, etc.) dissolved or dispersed in the water. Such a culture broth has composition prepared to allow good growth of the microorganism.

Examples of the organic substance include alcohols, such as ethanol and 2,3-butanediol, acetic acid, lactic acid, isoprene, and the like. Among all, an organic substance containing alcohol (particularly, ethanol) is preferred. Ethanol may be used as fuel ethanol as well as, for example, a raw material for cosmetics, beverages, chemical substances, fuel (jet fuel), and the like and additives for foods and the like, and thus has extremely high versatility.

The microorganism in the present invention is not particularly limited as long as having capability of generating an organic substance from gas, such as carbon monoxide and hydrogen. Particularly due to the high performance of generating an organic substance from gas, the microorganism preferably contains a bacterium growing on gas.

Such a bacterium growing on gas includes both a eubacterium and an archaebacterium.

Examples of the eubacterium include a Clostridium bacterium, a Moorella bacterium, an Acetobacterium bacterium, a Carboxydocella bacterium, a Rhodopseudomonas bacterium, a Eubacterium bacterium, a Butyribacterium bacterium, an Oligotropha bacterium, a Bradyrhizobium bacterium, a Ralsotonia bacterium as an aerobic hydrogen oxidizing bacterium, and the like.

Meanwhile, examples of the archaebacterium include a Methanobacterium bacterium, a Methanobrevibacter bacterium, a Methanocalculus bacterium, a Methanococcus bacterium, a Methanosarcina bacterium, a Methanosphaera bacterium, a Methanothermobacter bacterium, a Methanothrix bacterium, a Methanoculleus bacterium, a Methanofollis bacterium, a Methanogenium bacterium, a Methanospirillium bacterium, a Methanosaeta bacterium, a Thermococcus bacterium, a Thermofilum bacterium, an Arcaheoglobus bacterium, and the like.

Among them, preferred archaebacteria are a Methanosarcina bacterium, a Methanococcus bacterium, a Methanothermobacter bacterium, a Methanothrix bacterium, a Thermococcus bacterium, a Thermofilum bacterium, and an Archaeoglobus bacterium.

Moreover, due to the excellent capability of growing on carbon monoxide and carbon dioxide, preferred archaebacteria are a Methanosarcina bacterium, a Methanothermobactor bacterium, and a Methanococcus bacterium, and particularly preferred archaebacteria are a Methanosarcina bacterium and a Methanococcus bacterium.

Specific examples of the Methanosarcina bacterium include Methanosarcinabarkeri, Methanosarcina mazei, Methanosarcinaacetivorans, and the like.

From the bacteria growing on gas as mentioned above, a bacterium with high performance of generating an intended organic substance is selected to be used.

For instance, examples of the bacterium growing on gas with high performance of generating ethanol include Clostridium autoethanogenum, Clostridium ljungdahlii, Clostridiumaceticum, Clostridiumcarboxidivorans, Moorella thermoacetica, Acetobacterium woodii, and the like. Among all, from the perspective of the rate of the capability of growing on gas and the stability of culture, the bacterium growing on gas preferably contains a Clostridium bacterium and more preferably contains Clostridium autoethanogenum.

As the fermenter 4, it is possible to use, for example, a fermenter to stir the culture broth with stirring blades, a fermenter to stir the culture broth by circulating the culture broth itself, a fermenter to stir the culture broth by a water flow associated with a bubble flow occurred by ventilation of the supplied gas, and the like.

The gas purification section 3 may be provided as needed and may be omitted. That is, the gas outlet of the gasification furnace 2 may be directly connected to the gas inlet of the fermenter 4 via the gas line 101.

The fermenter 4 has a liquid outlet connected, via a liquid line 201, to a liquid inlet of the separation section 5. An organic substance is generated in the culture broth from gas in the fermenter 4, and the culture broth containing the organic substance and the microorganism is supplied to the separation section 5 as a discharge fluid by a pump, not shown.

The fermenter 4 is provided with a concentration sensor (not shown) to allow detection of the concentration of the organic substance (e.g., ethanol) in the culture broth (discharge fluid). For example, in a case of ethanol as the organic substance, the concentration of the organic substance in the culture broth is approximately from 1% to 10% in general.

The separation section 5 has a function of separating the discharge fluid (culture broth) discharged from the fermenter 4 into a first liquid that contains solid contents containing the microorganism and the organic substance (culture broth with concentrated bacteria) and a second liquid containing the organic substance and containing a less content of the solid substance than that in the first liquid. In the second liquid, the solid substance is not substantially contained or, even if contained, the solid substance is in an extremely trace amount.

Such a separation section 5 may be configured with, for example, a device provided with a filter, a device using centrifugation, a device using solution precipitation, a filtering device using sand, or the like. Among all, the separation section 5 is preferably configured with a device provided with a filter. The separation section 5 provided with a filter allows separation of the discharge fluid discharged from the fermenter 4 into the first liquid and the second liquid readily and at relatively low costs.

As such a filter, either a cross flow filter (so-called a "cross filter") or a full flow filter may be used and a cross filter is preferably used. Use of such a cross filter allows preferred preventing the filter from clogging with the solid contents.

The filter has a pore diameter that is appropriately set in accordance with the size of microorganism and thus not particularly limited, whereas the filter to be applied preferably has a pore diameter from submicron to 100 microns. The filter having a pore diameter of such a size allows more secure inhibition of passage of the microorganism without reducing the efficiency of passage of the second liquid.

The filter (porous membrane) may be configured with, for example: a ceramics material containing aluminum and silicon atoms; a resin material, such as a fluorine-based resin; a metal material, such as stainless steel; or the like. Among all, the filter is preferably configured with a fluorine-based resin or a ceramics material. Microorganisms are less likely to be attached (adsorbed) to such a filter and clogging is thus prevented, resulting in higher efficiency of separating the first liquid (concentrated broth) from the second liquid (filtrate).

The separation section 5 is provided with two liquid outlets, one of which is connected to a liquid inlet of the evaporation section 6 via a liquid line 202 and the other is connected to a liquid inlet of the organic substance purification section 7 via a liquid line 203. The first liquid discharged from the separation section 5 is supplied to the evaporation section 6 by a pump, not shown, and the second liquid discharged from the separation section 5 is supplied to the organic substance purification section 7 by a pump, not shown.

In the evaporation section 6, a liquid component containing the organic substance is evaporated from the first liquid. The temperature to evaporate the liquid component is preferably from 10°C to 180°C approximately and more preferably from 70°C to 110°C approximately. When the temperature to evaporate the liquid component is from 10°C to 40°C, a method of bubbling carrier gas in the first liquid within the scope of not critically affecting the vacuum environment or distillation at a later stage is preferred, and when the temperature exceeds 40°C, a method of distilling by heating is preferred.

Such an evaporation section 6 may be configured with, for example, an evaporation boiler, a distillation column, or the like.

In this situation, all the liquid component containing the organic substance may be evaporated or the intended organic substance may be preferentially evaporated. The former case allows a greater increase in the yield of the organic substance, and the latter case causes a paste residue containing part of the liquid component to be remained in the evaporation section 6 and thus facilitates an operation of removing the residue.

The moisture content in the residue discharged as a liquid, a solid, or a mixture thereof from the evaporation section 6 is preferably 90% or less of the water content in the first liquid supplied to the evaporation section 6, more preferably 75% or less, even more preferably 50% or less, and particularly preferably 25% or less, and may be even 0%. The residue with such a moisture content is capable of being used to generate gas without an additional auxiliary fuel when returned into the gasification furnace 2 and combusting in air or oxygen without an auxiliary fuel.

In the present embodiment, all or part of the residue discharged from the evaporation section 6 may be supplied to the gasification furnace 2. This allows reuse of the residue as part of the gas material. When the residue is a liquid or a mixture of a liquid and a solid, a line may be provided to connect the evaporation section 6 with the gasification furnace 2 to supply the residue from the evaporation section 6 via the line to the gasification furnace 2. When the residue is a solid, the residue may be manually (by machine) collected and supplied from the evaporation section 6 to the gasification furnace 2.

The evaporation section 6 has a gas outlet connected, via a gas line 103, to a gas inlet of the organic substance purification section 7. The gas discharged from the evaporation section 6 (vapor of the liquid component vaporized in the evaporation section 6) is supplied to the organic substance purification section 7 by a pump, not shown.

In the organic substance purification section 7, the organic substance is purified from the second liquid supplied from the separation section 5 and the gaseous liquid component supplied from the evaporation section 6. The gaseous liquid component may be partially liquefied.

Examples of the organic substance purification section 7 include a distillation column, a treatment device containing a pervaporation membrane, a treatment device containing a zeolite dehydration membrane, a treatment device to remove low boiling substances having a boiling point lower than the organic substance, a treatment device to remove high boiling substances having a boiling point higher than the organic substance, a treatment device containing an ion exchange membrane, and the like. Among all, the organic substance purification section 7 is preferably configured with a distillation column (still). Such a distillation column allows purification and recovery of a desired organic substance with high purity. According to the present invention, microorganisms are removed from the second liquid and the liquid component supplied to the distillation column and it is thus possible to prevent foaming in the distillation column and smoothly purify the organic substance.

The temperature in the distillation column during distillation of the organic substance (particularly ethanol) is preferably, but not particularly limited to, 100°C or less and more preferably from 70°C to 95°C approximately. Setting of the temperature in the distillation column in the above range allows more secure separation of the desired organic substance from other components, that is, purification of the organic substance.

The pressure in the distillation column during distillation of the organic substance may be normal pressure and is preferably less than atmospheric pressure and more preferably from 60 to 95 kPa (gauge pressure) approximately. Setting of the pressure in the distillation column in the above range allows improvement in efficiency of organic substance purification, leading to improvement in the yield of the organic substance.

In this situation, during distillation of the organic substance, gas is generated that contains hydrogen and carbon monoxide dissolved in the filtrate (culture broth). Supply of such gas to the gasification furnace 2 as flue gas also allows effective use of the flue gas generated in the distillation column for combustion of the gas material. At this point, the organic components, such as ethanol, contained in the gas is preferably removed by pressurization, cooling, or adsorption not for combustion but for recovery.

To the distillation column, the second liquid from the separation section 5 and the gas from the evaporation section 6 may be supplied from the same inlet or different inlets that are vertically separate. When the second liquid from the separation section 5 and the gas from the evaporation section 6 are supplied from different inlets, the second liquid may be supplied from an upper inlet (liquid inlet) and the gas from a lower inlet (vapor inlet) or the gas may be supplied from an upper inlet (vapor inlet) and the second liquid from a lower inlet (liquid inlet), where the former case is preferred.

A distillation column configured to have the second liquid supplied from the upper inlet and the gas supplied from the lower inlet allows inhibition of the amount of thermal energy (amount of used vapor) used for purification of the organic substance.

In this case, the number of stages in the distillation column is, but not particularly limited to, preferably five or more, more preferably ten or more, and even more preferably from 10 to 20 approximately. Such configuration allows a greater reduction in the amount of the thermal energy used for purification of the organic substance without causing an increase in size of the distillation column. The number of stages indicates an amount of packing equivalent to the intended number of stages for a still with a continuous stage, such as one filled with irregular packing.

Although the present configuration tends to cause a large amount of ethanol to be contained in the bottoms from the distillation column and a decrease in the ethanol recovery, use of all or part of the bottoms as a culture broth (return to the culture vessel) tends to allow inhibition of a decrease in the overall ethanol recovery.

The device for producing an organic substance and the gas treatment system of the present invention have been described above while the present invention is not limited to the above description. For example, the device for producing an organic substance and the gas treatment system of the present invention may include respective other optional configurations or may have optional configuration as a substitution exhibiting a similar function.

Examples

The present invention is then described with specific Examples. It should be noted that the present invention is not limited to the following specific Examples.

Comparative Example 1

In the gas treatment system 1 illustrated in Fig. 1, the separation section 5 and the evaporation section 6 were omitted and the culture broth (broth) discharged from the culture vessel 4 was directly supplied to the distillation column (organic substance purification section 7).

In the culture vessel 4, Clostridium autoethanogenum was used as the microorganism (bacterium growing on gas) to generate ethanol from gas derived from MSW.

As a result, foaming occurred in the distillation column and thus the operation of the gas treatment system 1 was stopped.

Comparative Example 2

In the gas treatment system 1 illustrated in Fig. 1, the evaporation section 6 was omitted and only a filtrate (second liquid) obtained by separating the culture broth (broth) discharged from the culture vessel 4 with the separation section 5 provided with a ceramics filter with a pore diameter of 0.2 $\mu$m was supplied to the distillation column (organic substance purification section 7).

In the culture vessel 4, Clostridium autoethanogenum was used as the microorganism (bacterium growing on gas) to generate ethanol from gas derived from MSW.

As a result, although the distillation itself in the distillation column had no problem, the filtrate was obtained only in an amount 75% of the culture broth discharged from the culture vessel 4 and thus it was not possible to recover a sufficient amount of ethanol.

Investigation of Injection (Supply) Site of Second Liquid from Separation Section 5 and Vapor from Evaporation Section 6 to Distillation Column

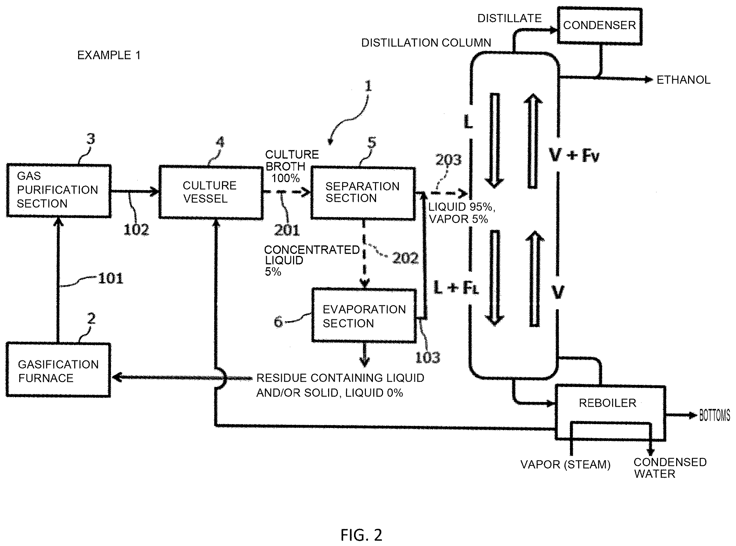

A description is given to, in the gas treatment systems illustrated in Figs. 2 (Example 1) and 3 (Example 2), a simulated value (calculated from a McCabe-Thiele diagram) of the amount of thermal energy (amount of used vapor) used for purification of ethanol in the distillation column.

In this context, it was assumed that only water ($H_2O$) and ethanol ($C_2H_6O$) were contained in the second liquid from the separation section 5 supplied via the liquid line 203 (hereinafter, referred to as "liquid Feed") and the vapor from the evaporation section 6 supplied via the gas line 103 (hereinafter, referred to as "vaporous Feed") and that a molar fraction of ethanol (number of ethanol molecules/( number of ethanol molecules + number of water molecules)) in each Feed was defined as 0.018519 (46 g/kg at ethanol mass concentration).

It was also assumed that distillation in the distillation column was to recover the bottoms with a molar fraction of ethanol of 0.000926 (2.3 g/kg at ethanol mass concentration) and the distillate with a molar fraction of ethanol of 0.5 (719 g/kg at ethanol mass concentration).

In both Examples 1 and 2, each Feed was injected into the stage where the liquid-gas concentration in the distillation column substantially coincides with the liquid-gas concentration of the Feed. For example, when the distillation column has ten stages, the mixture of the liquid Feed and the vaporous Feed was injected between the eighth stage and the nineth stage in Example 1, and the liquid Feed was injected between the eighth stage and the nineth stage and the vaporous Feed was injected into the second stage in Example 2.

In the simulation, the total number of moles per unit time in the liquid Feed was defined as "1" and the total number of moles per unit time in the vaporous Feed was defined as 5%/95% ≈ 0.053.

In each Example, ethanol was purified only to a concentration of less than 80%. The amount of used vapor (amount of steam) was determined only considering the amount used for evaporation (latent heat), and the evaporation latent heat per mole was approximated to be equal for water and ethanol.

The used vapor was assumed to be converted at a molar ratio of 1:1 to vapor of the target substance in the reboiler and the evaporation section 6. This is a reasonable assumption because the evaporation latent heat per mole of ethanol (38.6 kJ/mol (78°C)) is close to that of water (41.6 kJ/mol (78°C)).

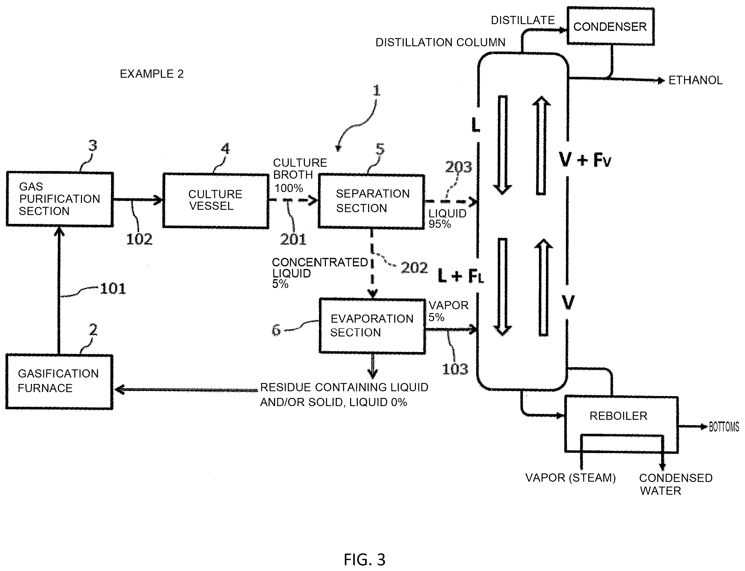

In Figs. 2 and 3, "liquid 0%" means to contain 0% of moisture and a liquid component, such as butanediol and acetic acid, sometimes remains.

Calculation Method in Example 1

Each variable denotes the following meaning.

L: flow rate (mol/hr) of liquid components of water and ethanol falling from the condenser.
V: flow rate (mol/hr) of vapor components of water and ethanol generated by heating of the reboiler.
$F_L$: flow rate of liquid components of water and ethanol in Feed.
$F_V$: flow rate of vapor components of water and ethanol in Feed.
xfeed: molar fraction of ethanol in the culture broth (except solid components) discharged from the culture vessel 4.
xf: molar fraction of ethanol in the liquid component at the injection point.
yf: molar fraction of ethanol in the vapor component at the injection point.
xtop: molar fraction of ethanol in the liquid component in the condenser.
ytop: molar fraction of ethanol in the vapor component in the condenser.
xbtm: molar fraction of ethanol in the liquid component in the reboiler.
ytop: molar fraction of ethanol in the vapor component in the reboiler.
m1: slope of the lower part (from the injection point to the reboiler) of the operating line.
m2: slope of the upper part (from the injection point to the condenser) of the operating line.
a: ratio of the vapor component to the liquid component in Feed.

Seven variables (L, V, a, $F_V$, sf, yf, and m1) are defined as dependent variables and the following seven equations are established with them.

Note that $F_L$ = 1, which is normalized.

$$\text{Equation 1: } m2 * (xf - xtop) - (yf - ytop) = 0$$

$$\text{Equation 2: } m1 * (xf - xbtm) - (yf - ybtm) = 0$$

$$\text{Equation 3: } -a + (xfeed - xf) / (xfeed - yf) = 0$$

$$\text{Equation 4: } m2 - L / (F_V + V) = 0$$

$$\text{Equation 5: } m1 - (F_L + L)/ V = 0$$

$$\text{Equation 6: } -a + F_V / F_L = 0$$

$$\text{Equation 7: } F_L - 1 = 0$$

Here, although m2 is an independent variable, careless setting of this value causes steps of the evaporation stages, which are drawn from the lower part in the McCabe-Thiele diagram, not to pass through xtop and ytop. In this example, m2 was varied at intervals of 0.0002 and the case where the step line runs closest to, but not exceeding, xtop and ytop was defined as m2 at each stage.

Solving the above equations causes the amount of used vapor (amount of steam) to become a total ($F_V$ + V) of an amount of heating by the reboiler at the bottom of the distillation column and an amount of heating by the evaporation section 6, and the amount of used vapor per liquid Feed becomes ($F_V$ + V) / $F_L$. This value was plotted on the ordinate as the number of moles of the used vapor.

Calculation Method in Example 2

Each variable denotes the following meaning.

L: flow rate (mol/hr) of liquid components of water and ethanol falling from the condenser.
V: flow rate (mol/hr) of vapor components of water and ethanol generated by heating of the reboiler.
$F_L$: flow rate of liquid components of water and ethanol in Feed.
$F_V$: flow rate of vapor components of water and ethanol in Feed.
xfeed: molar fraction of ethanol in the culture broth (except solid components) discharged from the culture vessel 4.
xlf: molar fraction of ethanol in the liquid component at the upper injection point = xfeed.
ylf: molar fraction of ethanol in the vapor component at the upper injection point.
xvf: molar fraction of ethanol in the liquid component at the lower injection point.
yvf: molar fraction of ethanol in the vapor component at the lower injection point = xfeed.
xtop: molar fraction of ethanol in the liquid component in the condenser.
ytop: molar fraction of ethanol in the vapor component in the condenser.
xbtm: molar fraction of ethanol in the liquid component in the reboiler.
ytop: molar fraction of ethanol in the vapor component in the reboiler.
m1: slope of the lower part (from the lower injection point to the reboiler) of the operating line.
m2: slope of the intermediate part (from the lower injection point to the upper injection point) of the operating line.
m3: slope of the upper part (from the upper injection point to the condenser) of the operating line.
a: ratio of the vapor component to the liquid component in Feed.

Eight variables (L, V, a, $F_V$, ylf, xvf, m1, and m3) are defined as dependent variables and the following eight equations are established with them.
Note that $F_L$ = 1, which is normalized.

$$\text{Equation 1: m3} * (xlf - xtop) - (ylf - ytop) = 0$$

$$\text{Equation 2: m2} * (xfeed - xvf) - (ylf - xfeed) = 0$$

$$\text{Equation 3: m1} * (xvf - xbtm) - (xfeed - ybtm) = 0$$

$$\text{Equation 4: m1} - (F_L + L) / V = 0$$

$$\text{Equation 5: m2} - (F_L + L) / (F_V + V) = 0$$

$$\text{Equation 6: m3} - L / (F_V + V) = 0$$

$$\text{Equation 7: a} - F_V / F_L = 0$$

$$\text{Equation 8: } F_L - 1 = 0$$

Here, although m2 is an independent variable, careless setting of this value causes steps of the evaporation stages, which are drawn from the lower part in the McCabe-Thiele diagram, not to pass through xtop and ytop. In this example, m2 was varied at intervals of 0.0002 and the case where the step line runs closest to, but not exceeding, xtop and ytop was defined as m2 at each stage.
Solving the above equations causes the amount of used vapor (amount of steam) to become a total ($F_V$ + V) of an amount of heating by the reboiler at the bottom of the distillation column and an amount of heating by the evaporation section 6, and the amount of used vapor per liquid Feed becomes ($F_V$ + V) / $F_L$. This value was plotted on the ordinate as the number of moles of the used vapor.

The results of the above simulation are shown in Table 1 below.

It should be noted that, for comparison under the identical conditions, the ethanol concentrations of the bottoms from the still were coincided with each other. This indicates that the increase in the outflow of ethanol to the bottom side in Example 2 was not in the range causing a problem.

[Table 1]

Number of Stages in Distillation Column vs Amount of Used Vapor
(Liquid Feed = 1, Vaporous Feed = 0.053)

Number of Stages in Distillation Column

As a result of the simulation, it was found that the amount of used vapor in Example 2 was below the amount of used vapor in Example 1 when the number of stages in the still was five or more.

Reference Signs List

| 1 | Gas Treatment System |
| 2 | Gasification Furnace |
| 3 | Gas Purification Section |
| 4 | Culture Vessel |
| 5 | Separation Section |
| 6 | Evaporation Section |
| 7 | Organic Substance Purification Section |
| 101 | Gas Line |
| 102 | Gas Line |
| 103 | Gas Line |
| 201 | Liquid Line |
| 202 | Liquid Line |
| 203 | Liquid Line |

**Claims**

1. A device for producing an organic substance, comprising:

a fermenter (4) configured to generate an organic substance from gas by fermenting action of a microorganism;
a separation section (5) configured to separate a discharge fluid discharged from the fermenter into a first liquid

9

containing a solid substance containing the microorganism and the organic substance and a second liquid containing the organic substance, and the solid substance is not substantially contained in the second liquid;

an evaporation section (6) configured to evaporate a liquid component containing the organic substance from the first liquid; and

a purification section (7) configured to purify the organic substance from the second liquid and the liquid component vaporized in the evaporation section,

wherein the purification section is configured with a distillation column provided with a liquid inlet configured to supply the second liquid and a vapor inlet provided below the liquid inlet and configured to supply vapor as the liquid component vaporized in the evaporation section.

2. The device for producing an organic substance according to Claim 1, wherein the distillation column has number of stages of five or more.

3. The device for producing an organic substance according to Claim 1 or 2, wherein the microorganism includes a bacterium growing on gas.

4. The device for producing an organic substance according to Claim 3, wherein the bacterium growing on gas includes a Clostridium bacterium.

5. The device for producing an organic substance according to any one of Claims 1 through 4, wherein the organic substance includes ethanol.

6. The device for producing an organic substance according to any one of Claims 1 through 5, wherein the gas is generated by combusting a waste.

7. The device for producing an organic substance according to any one of Claims 1 through 6, wherein the separation section is provided with a filter.

8. The device for producing an organic substance according to Claim 7, wherein the filter has a pore diameter from 0.1 to 100 $\mu$m.

9. A gas treatment system (1), comprising:

a gas generation section (2) configured to generate gas by combusting, heating, or partially oxidizing a gas material; and

the device for producing an organic substance according to any one of Claims 1 through 8, the device configured to obtain an organic substance using the gas generated in the gas generation section.

10. The gas treatment system (1) according to Claim 9, wherein the gas generation section (2) and the evaporation section (5) are configured to supply at least part of a residue discharged as a liquid, a solid, or a mixture thereof from the evaporation section to the gas generation section.


**Patentansprüche**

1. Vorrichtung zur Herstellung einer organischen Substanz, umfassend:

einen Fermenter (4), der so konfiguriert ist, dass er durch die Fermentationswirkung eines Mikroorganismus aus Gas eine organische Substanz erzeugt;

einen Trennabschnitt (5), der so konfiguriert ist, dass er eine aus dem Fermenter abgeleitete Flüssigkeit in eine erste Flüssigkeit, die einen Feststoff enthält, der den Mikroorganismus und die organische Substanz enthält, und eine zweite Flüssigkeit, die die organische Substanz enthält, trennt, wobei der Feststoff in der zweiten Flüssigkeit nicht im Wesentlichen enthalten ist;

einen Verdampfungsabschnitt (6), der so konfiguriert ist, dass er eine die organische Substanz enthaltende flüssige Komponente aus der ersten Flüssigkeit verdampft; und

einen Reinigungsabschnitt (7), der so konfiguriert ist, dass er die organische Substanz aus der zweiten Flüssigkeit und der in der Verdampfungsstufe verdampften flüssigen Komponente reinigt,

wobei der Reinigungsabschnitt mit einer Destillationskolonne ausgestattet ist, die über einen Flüssigkeitseinlass,

# EP 3 778 852 B1

der so konfiguriert ist, dass er die zweite Flüssigkeit zuführt, und einen unter dem Flüssigkeitseinlass liegenden Dampfeinlass, der so konfiguriert ist, dass er Dampf als die in der Verdampfungsstufe verdampfte Flüssigkeitskomponente zuführt, verfügt.

2. Vorrichtung zur Herstellung einer organischen Substanz nach Anspruch 1, wobei die Destillationskolonne fünf oder mehr Stufen aufweist.

3. Vorrichtung zur Herstellung einer organischen Substanz nach Anspruch 1 oder 2, wobei der Mikroorganismus ein auf Gas wachsendes Bakterium umfasst.

4. Vorrichtung zur Herstellung einer organischen Substanz nach Anspruch 3, wobei das auf Gas wachsende Bakterium ein Clostridium-Bakterium umfasst.

5. Vorrichtung zur Herstellung einer organischen Substanz nach einem der Ansprüche 1 bis 4, wobei die organische Substanz Ethanol umfasst.

6. Vorrichtung zur Herstellung einer organischen Substanz nach einem der Ansprüche 1 bis 5, wobei das Gas durch Verbrennung eines Abfalls erzeugt wird.

7. Vorrichtung zur Herstellung einer organischen Substanz nach einem der Ansprüche 1 bis 6, wobei der Trennabschnitt mit einem Filter versehen ist.

8. Vorrichtung zur Herstellung einer organischen Substanz nach Anspruch 7, wobei der Filter einen Porendurchmesser von 0,1 bis 100 $\mu$m aufweist.

9. Gasbehandlungssystem (1), umfassend:

einen Gaserzeugungsabschnitt (2), der so konfiguriert ist, dass er durch Verbrennung, Erhitzen oder teilweises Oxidieren eines gasförmigen Materials Gas erzeugt; und
die Vorrichtung zur Herstellung einer organischen Substanz nach einem der Ansprüche 1 bis 8, wobei die Vorrichtung so konfiguriert ist, dass sie eine organische Substanz unter Verwendung des im Gaserzeugungsabschnitt erzeugten Gases gewinnt.

10. Gasbehandlungssystem (1) nach Anspruch 9, wobei der Gaserzeugungsabschnitt (2) und der Verdampfungsabschnitt (5) so konfiguriert sind, dass sie mindestens einen Teil eines als Flüssigkeit, Feststoff oder Gemisch davon aus dem Verdampfungsabschnitt austretenden Rückstands dem Gaserzeugungsabschnitt zuführen.

**Revendications**

1. Dispositif de production d'une substance organique, comportant :

un fermenteur (4) configuré pour générer une substance organique à partir d'un gaz par une action de fermentation d'un micro-organisme ;
une section de séparation (5) configurée pour séparer un fluide d'évacuation évacué du fermenteur en un premier liquide contenant une substance solide contenant le micro-organisme et la substance organique, et un second liquide contenant la substance organique, et la substance solide n'est pratiquement pas contenue dans le second liquide ;
une section d'évaporation (6) configurée pour évaporer un composant liquide contenant la substance organique à partir du premier liquide ; et
une section de purification (7) configurée pour purifier la substance organique provenant du second liquide et le composant liquide vaporisé dans la section d'évaporation,
dans lequel la section de purification est configurée avec une colonne de distillation pourvue d'une entrée de liquide configurée pour fournir le second liquide, et d'une entrée de vapeur agencée sous l'entrée de liquide et configurée pour fournir de la vapeur en tant que composant liquide vaporisé dans la section d'évaporation.

2. Dispositif de production d'une substance organique selon la revendication 1, dans lequel la colonne de distillation a un nombre d'étages égal à cinq ou plus.

**3.** Dispositif de production d'une substance organique selon la revendication 1 ou 2, dans lequel le micro-organisme inclut une bactérie se développant sur un gaz.

**4.** Dispositif de production d'une substance organique selon la revendication 3, dans lequel la bactérie se développant sur un gaz inclut une bactérie Clostridium.

**5.** Dispositif de production d'une substance organique selon l'une quelconque des revendications 1 à 4, dans lequel la substance organique inclut de l'éthanol.

**6.** Dispositif de production d'une substance organique selon l'une quelconque des revendications 1 à 5, dans lequel le gaz est généré en brûlant un déchet.

**7.** Dispositif de production d'une substance organique selon l'une quelconque des revendications 1 à 6, dans lequel la section de séparation est pourvue d'un filtre.

**8.** Dispositif de production d'une substance organique selon la revendication 7, dans lequel le filtre a un diamètre de pores de 0,1 à 100 $\mu$m.

**9.** Système de traitement de gaz (1), comportant :

une section de génération de gaz (2) configurée pour générer du gaz par combustion, chauffage ou oxydation partielle d'une matière gazeuse ; et
le dispositif de production d'une substance organique selon l'une quelconque des revendications 1 à 8, le dispositif étant configuré pour obtenir une substance organique en utilisant le gaz généré dans la section de génération de gaz.

**10.** Système de traitement de gaz (1) selon la revendication 9, dans lequel la section de génération de gaz (2) et la section d'évaporation (5) sont configurées pour fournir à la section de génération de gaz au moins une partie d'un résidu évacué sous forme de liquide, de solide ou d'un mélange de ceux-ci à partir de la section d'évaporation.

FIG. 1

FIG. 2

FIG. 3

EXAMPLE 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015077120 A
- JP 2016029921 A
- US 2009215139 A